(19) Europäisches Patentamt
European Patent Office
Office européen des brevets

(11) **EP 1 211 942 B1**

(12) **EUROPÄISCHE PATENTSCHRIFT**

(45) Veröffentlichungstag und Bekanntmachung des Hinweises auf die Patenterteilung:
**02.04.2003 Patentblatt 2003/14**

(21) Anmeldenummer: **00966031.7**

(22) Anmeldetag: **15.09.2000**

(51) Int Cl.[7]: **A01N 59/12**

(86) Internationale Anmeldenummer:
**PCT/EP00/09051**

(87) Internationale Veröffentlichungsnummer:
**WO 01/019191 (22.03.2001 Gazette 2001/12)**

(54) **VERFAHREN ZUR HERSTELLUNG VON POLYVINYLPYRROLIDON-IOD IN WÄSSRIGER LÖSUNG**

METHOD FOR THE PRODUCTION OF POLYVINYLPYRROLIDONE-IODINE IN AN AQUEOUS SOLUTION

PROCEDE POUR PRODUIRE UN COMPLEXE POLYVINYLPYRROLIDONE-IODE EN SOLUTION AQUEUSE

(84) Benannte Vertragsstaaten:
**AT BE CH CY DE DK ES FI FR GB GR IE IT LI LU MC NL PT SE**
Benannte Erstreckungsstaaten:
**RO SI**

(30) Priorität: **16.09.1999 DE 19944464**

(43) Veröffentlichungstag der Anmeldung:
**12.06.2002 Patentblatt 2002/24**

(73) Patentinhaber: **BASF AKTIENGESELLSCHAFT**
**67056 Ludwigshafen (DE)**

(72) Erfinder:
• **WITTELER, Helmut**
**67157 Wachenheim (DE)**
• **SANNER, Axel**
**67227 Frankenthal (DE)**

(74) Vertreter: **Kinzebach, Werner, Dr. et al**
**Reitstötter, Kinzebach & Partner,**
**Postfach 21 11 60**
**67011 Ludwigshafen (DE)**

(56) Entgegenhaltungen:
**EP-A- 0 027 613**

**Beschreibung**

Die vorliegende Erfindung betrifft ein Verfahren zur Herstellung von Polyvinylpyrrolidon-Iod (PVP-Iod) in wässriger Lösung. Polyvinylpyrrolidon-Iod ist das Reaktionsprodukt von Polyvinylpyrrolidon (PVP) mit elementarem Iod, das in steigendem Maße wegen seiner germiziden, bakteriziden, fungiziden und desinfizierenden Eigenschaften verwendet wird.

Verfahren zur Herstellung von PVP-Iod sind grundsätzlich bekannt. Die Herstellung erfolgt in der Regel durch Vermischen von Polyvinylpyrrolidon als Feststoff oder Lösung mit Iod oder Iod-Verbindungen. Problematisch ist jedoch die Herstellung von PVP-Iod mit guter Stabilität bei guter Verfügbarkeit des enthaltenen Iods.

Maß für die Stabilität eines PVP-Iods ist zum einen der Verteilungskoeffizient (VK) des Iods zwischen einer wässrigen PVP-Iod-Lösung und Heptan. Dieser wird gemäß US-A-3,028,300 durch einminütiges intensives Schütteln einer Iodlösung mit einem verfügbaren Iodgehalt von 1,0 % mit Heptan bei 25 °C bestimmt und liegt bei einer hinreichend festen Bindung zwischen Polyvinylpyrrolidon und Iod bei etwa 200 und darüber. Zum anderen wird als Maß für die Stabilität, insbesondere die Lagerstabilität des PVP-Iods der Iodverlust, d. h. die prozentuale Abnahme des verfügbaren Iods, beim Erwärmen über einen definierten Zeitraum bestimmt.

Eine wichtige Kenngröße für die Stabilität von PVP-Iod ist das Iod:Iodid-Verhältnis. Bei einem Iod:Iodid-Verhältnis von etwa 2:1 ist in der Regel die Verbindung von PVP und Iod so stark, dass kein Iodgeruch mehr wahrnehmbar ist und ein um den Gasraum über der Probe eingebrachtes feuchtes Kaliumiodid-Stärkepapier nicht gefärbt wird.

Eine Reihe von Publikationen, z. B. die DE-PS-10 37 075, die US-A-2,900,305, die US-A-2,826,532, die US-A-3,028,300, die US-A-3,898,326 und die DE-AS-24 39 197, beschreiben Maßnahmen, die PVP-Iod mit verbesserten Eigenschaften bereitstellen sollen. Die vorgenannten Verfahren führen jedoch entweder zu PVP-Iod-Komplexen mit unbefriedigender Stabilität oder benötigen für die Ausbildung einer hinreichend festen Bindung zwischen PVP und Iod eine sogenannte Temperung, d. h. ein anschließendes Erhitzen des zuvor gebildeten PVP-Iods auf Temperaturen von 70 bis 90 °C, die in der Regel über 10 Stunden, in den meisten Fällen im Bereich von 20 bis 64 Stunden dauert. Zur Erreichung akzeptabler Herstellungszeiten muss das Tempern bei vergleichsweise hohen Temperaturen, d. h. oberhalb von 70 °C durchgeführt werden. Das Tempern pulverförmigen PVP-Iods ist technisch sehr aufwendig, da das Pulver bei diesen Temperaturen stark zum Verklumpen neigt. Wird das Tempern dagegen in wässriger Lösung durchgeführt, beobachtet man bei den erforderlichen Temperaturen in der Regel einen hohen Iodverlust durch Bildung eines Bodensatzes und/oder durch Sublimation.

Die DE-A-25 40 170 beschreibt die Verwendung eines Polyvinylpyrrolidon und Alkali-Iodiden als Iodionen liefernde Verbindung, die ohne langes Tempern zu PVP-Iod befriedigender stbilität führen. Die resultierenden PVP-Iod-Lösungen dürfen jedoch wegen ihres Aschegehaltes nicht für pharmazeutische Präparate verwendet werden.

Die EP-A-027 613 beschreibt ein Verfahren zur Herstellung von PVP-Iod, wobei man PVP, elementares Iod und eine die Iodidbildung beschleunigende Verbindung in wässriger Lösung umsetzt. Die Gesamtreaktionszeit, d. h. die als Reaktionszeit bezeichnete und die zum Tempern benötigte Zeit, liegt trotz des Einsatzes der die Iodidbildung beschleunigenden Verbindung im Bereich von 7 bis 32 Stunden, wobei auch nach diesem Verfahren eine Gesamtreaktionszeit von mindestens 14 Stunden notwendig ist, um einen Iodverlust von unter etwa 7 % zu erreichen. Als PVP-Lösungen werden solche mit einer Konzentration von 50 Gew.-% und einem R-Wert von 13, einer Konzentration von 40 Gew.-% und einem K-Wert von 17 und einer Konzentration von 30 Gew.-% und einem K-Wert von 32 eingesetzt.

Aufgabe der vorliegenden Erfindung ist es daher, ein Verfahren bereitzustellen, das eine einfache und schnelle Herstellung von PVP-Iod, ausgehend von elementarem Iod, ermöglicht. Darüber hinaus soll PVP-Iod bereitgestellt werden, das eine verbesserte Stabilität, insbesondere verbesserte Lagerstabilität, aufweist.

Erfindungsgemäß wird die Aufgabe durch ein Verfahren gelöst, bei dem das Polyvinylpyrrolidon in wässriger Lösung mit einer hohen Konzentration und vorzugsweise einem hohen R-Wert unter Verwendung von elementarem Iod zu Polyvinylpyrrolidon-Iod umgesetzt wird. Dabei war es überraschend, dass beim Einsatz von Polyvinylpyrrolidon-Lösungen in den erfindungsgemäßen Konzentrationen sich die Reaktionszeiten deutlich verkürzen und das gebildete PVP-Iod einen deutlich verringerten Iodverlust zeigt. Insbesondere war es überraschend, dass die PVP- und PVP-Iod-Lösungen auch bei hohen Konzentrationen und hohen K-Werten eine vergleichsweise niedrige Viskosität und keine oder nur geringe Viskositätssteigerungen während des Verfahrens zeigen, so dass eine einfache Durchführung des Verfahrens gewährleistet ist.

Gegenstand der vorliegenden Erfindung ist daher ein Verfahren zur Herstellung von Polyvinylpyrrolidon-Iod in wässriger Lösung, wobei man eine wässrige Polyvinylpyrrolidon-Lösung und wenigstens 4,0 Gew.-% elementares Iod, bezogen auf das als Feststoff gerechnete Polyvinylpyrrolidon, vermischt, dadurch gekennzeichnet, dass zum Zeitpunkt des Vermischens die Konzentration c des wässrigen Polyvinylpyrrolidons, bezogen auf die Gesamtmenge aus Polyvinylpyrrolidon und Wasser, und der K-Wert des Polyvinylpyrrolidons der folgenden Relation gehorcht:

$$c > 100 \times [0{,}1 + 8 : (K + 5)]$$

wobei c in Gew.-% angegeben ist und der K-Wert nach Fikentscher im Bereich von 10 bis 100 liegt. Die Konzentration c bezieht sich daher nur auf das Verhältnis von Polyvinylpyrrolidon zu Wasser.

Die mit dem erfindungsgemäßen Verfahren hergestellten Polyvinylpyrrolidon-Iod-Lösungen und das aus diesen durch Entfernen des Wassers und anderer flüchtiger Bestandteile erhältliche feste PVP-Iod weisen vorzugsweise ein Iod:Iodid-Verhältnis von etwa 2:1 und einen Verteilungskoeffizienten (VK), bestimmt gemäß US-A-3,028,300, im Bereich von 190 bis 250 auf. Das erfindungsgemäß erhältliche PVP-Iod weist vorzugsweise einen Iodverlust von < 6 %, bevorzugt < 5 % und besonders bevorzugt < 4 % auf, bestimmt an wässrigen Lösungen mit einem Gehalt an verfügbarem Iod von 1,0 % nach 15 Stunden Lagerung bei 80 °C.

In der oben angegebenen Relation stellt die Konzentration c eine vom R-Wert des eingesetzten Polyvinylpyrrolidons abhängige Mindestkonzentration der erfindungsgemäß zu verwendenden Lösungen dar. Vorzugsweise liegt die maximale Konzentration c der Polyvinylpyrrolidon-Lösung während des erfindungsgemäßen Verfahrens unterhalb von 90 Gew.-%, bevorzugt unter 85 Gew.-% und besonders bevorzugt unter 80 Gew.-%.

Bevorzugt gehorcht die Konzentration c des wässrigen Polyvinylpyrrolidons, bezogen auf die Gesamtmenge aus Polyvinylpyrrolidon und Wasser, und der K-Wert des Polyvinylpyrrolidons zum Zeitpunkt des Vermischens der Relation $c > 100 \times [0{,}1 + 8 : (K + 2)]$

Der K-Wert nach Fikentscher stellt ein Maß für das Molekulargewicht des Polyvinylpyrrolidons dar und wird gemäß H. Fikentscher, Cellulose-Chemie, 13, 38-64 und 71-74 (1932) als 1 gew.-%ige Lösung in Wasser bestimmt.

Für das erfindungsgemäße Verfahren sind Polyvinylpyrrolidone mit K-Werten im Bereich von 10 bis 100, vorzugsweise 20 bis 50 und besonders bevorzugt 25 bis 50, z. B. etwa 30 oder etwa 40 geeignet. Mit dem erfindungsgemäßen Verfahren können jedoch auch vorteilhaft Polyvinylpyrrolidone mit größeren K-Werten verarbeitet werden, z.B. Polyvinylpyrrolidone mit K-Werten von etwa 70 oder etwa 85.

Polyvinylpyrrolidone mit K-Werten im Bereich von 10 bis 20 werden vorzugsweise in einer Konzentration c im Bereich von 65 bis 90 Gew.-%, besonders bevorzugt 70 bis 85 Gew.-%, im erfindungsgemäßen Verfahren eingesetzt. Polyvinylpyrrolidone mit R-Werten im Bereich von > 20 bis 27 werden vorzugsweise in einer Konzentration c von 43 bis 80 Gew.-%, besonders bevorzugt 45 bis 70 Gew.-%, im erfindungsgemäßen Verfahren eingesetzt. Vorzugsweise setzt man im erfindugsgemäßen Verfahren eine Polyvinylpyrrolidon-Lösung eines Polyvinylpyrrolidons mit einem K-Wert > 27 und einem Polyvinylpyrrolidon-Gehalt von > 35 Gew.-% ein. Polyvinylpyrrolidone mit einem R-Wert im Bereich von 28 bis 50, z. B. etwa 30 oder etwa 40, werden bevorzugt in einer Konzentration c von 36 bis 75 Gew.-%, besonders bevorzugt 42 bis 65 Gew.-% und ganz besonders bevorzugt 45 bis 55 Gew.-%, z. B. etwa 45 Gew.-%, eingesetzt. Polyvinylpyrrolidone mit einem K-Wert im Bereich von > 50 bis 100, z. B. etwa 60, etwa 70 oder etwa 90, werden bevorzugt in einer Konzentration c von 18 bis 50 Gew.-%, besonders bevorzugt 19 bis 40 Gew.-% und ganz besonders bevorzugt 20 bis 35 Gew.-%, z. B. etwa 25 Gew.-%, eingesetzt.

Das im erfindungsgemäßen Verfahren eingesetzte Polyvinylpyrrolidon kann grundsätzlich auf beliebigem Weg erhalten werden. Für das erfindungsgemäße Verfahren geeignetes Polyvinylpyrrolidon kann beispielsweise durch Polymerisation in einem organischen Lösungsmittel, wie Isopropanol oder Toluol, unter Verwendung von Radikalbildnern, insbesondere organischen Per-Verbindungen, z. B. Dialkylperoxiden, oder Azoverbindungen, z. B. AIBN, hergestellt und gegebenenfalls anschließend einer Nachbehandlung, z. B. einer Wasserdampfdestillation, unterworfen werden. Das Polyvinylpyrrolidon kann beispielsweise auch durch radikalische Polymerisation in Wasser, gegebenenfalls im Gemisch mit organischen Lösungsmitteln in Gegenwart von wasserlöslichen Radikalbildnern, z. B. Wasserstoffperoxid oder Natriumperoxodisulfat, hergestellt werden. Eine Reihe von Verfahren zur Herstellung von Polyvinylpyrrolidon sind dem Fachmann bekannt und beispielsweise in Houben-Weyl, Methoden der Organischen Chemie, Georg Thieme-Verlag, Stuttgart, beschrieben.

Geeignet für das erfindungsgemäße Verfahren sind auch Polyvinylpyrrolidone, die nach der Polymerisation einem Hydrierungsprozess unterzogen werden. Dabei erfolgt die Hydrierung des Polyvinylpyrrolidons nach den üblichen, bekannten Verfahren. Eine Hydrierung mit Wasserstoff ist beispielsweise in der US-A-2,914,516 beschrieben. Ebenfalls geeignet sind Polyvinylpyrrolidone, die mit komplexen Hydriden behandelt wurden, die beispielsweise in der EP-A-027 613 beschrieben sind.

Die in der DE-AS-28 18 767, EP-A-027 613 beschriebenen Polyvinylpyrrolidone, die Verfahren zu ihrer Herstellung und geeignete Reaktanden und Reaktionsbedingungen können analog auch zur Herstellung der erfindungsgemäß eingesetzten, hochkonzentrierten Polyvinylpyrrolidon-Lösungen eingesetzt werden. Auf diese beiden Schriften wird hiermit ausdrücklich Bezug genommen.

Als weitere Iodquellen können neben elementarem Iod Iodide oder sonstige Iod liefernde Verbindungen eingesetzt werden, wobei, bezogen auf das als Feststoff gerechnete Polyvinylpyrrolidon, wenigstens 4,0 Gew.-%, insbesondere wenigstens 6,0 Gew.-% und besonders bevorzugt wenigstens 8,0 Gew.-% elementares Iod eingesetzt werden. In der Regel wird das elementare Iod in einer Menge im Bereich von 5 bis 35 Gew.-%, vorzugsweise 10 bis 30 Gew.-% und insbesondere 15 bis 25 Gew.-%, z. B. etwa 20 Gew.-%, eingesetzt. Als weitere Iodquellen geeignete verbindungen sind beispielsweise Jodwasserstoff, Alkali- und Erdalkaliiodide, insbesondere Alkaliiodide, z. B. Natriumiodid und Kaliumiodid, Polyiodide, insbesondere Alkalimetallpolyiodide, Ammoniumiodide, insbesondere Tetraalkylammoniumiodide, z. B. Tetramethylammoniumiodid, Phosphortriiodid, und organische Säureiodide, z. B. Acetyliodid. Vorzugsweise wird wenigstens 50 %, insbesondere wenigstens 75 % und besonders bevorzugt wenigstens 90 %, des im Polyvinylpyrrolidon-Iod enthaltenen Iods in Form von elementarem Iod eingesetzt. In einer bevorzugten Ausführungsform des erfindungsgemäßen Verfahrens wird als Iodquelle ausschließlich elementares Iod eingesetzt.

Beim erfindungsgemäßen Verfahren können die Reaktanden, d. h. Iod, Polyvinylpyrrolidon und gegebenenfalls Reduktionsmittel sowie gegebenenfalls Hilfsstoffe, unabhängig voneinander in Substanz, in Suspension oder Lösung zusammengegeben werden. Das Vermischen der Reaktanden zur Bildung des PVP-Iods wird bei einer Konzentration c, wie oben definiert, durchgeführt.

Beispielsweise kann beim erfindungsgemäßen Verfahren das Iod und gegebenenfalls weitere Iodquellen in einem Lösungsmittel, vorzugsweise Wasser, vorgelegt und gegebenenfalls erwärmt werden. Anschließend wird das Polyvinylpyrrolidon, vorzugsweise als wässrige Lösung, zugegeben und das Reaktionsgemisch vermischt. Das Vermischen der Reaktanden kann bei der gleichen Temperatur wie die Zugabe des Polyvinylpyrrolidons oder bei einer anderen, vorzugsweise einer einer höheren, Temperatur erfolgen.

Beim erfindungsgemäßen Verfahren wird vorzugsweise das Polyvinylpyrrolidon als wässrige Lösung vorgelegt und gegebenenfalls erwärmt. Anschließend wird Iod und gegebenenfalls weitere Iodquellen zugegeben und das Reaktionsgemisch vermischt. Die Zugabe des Iods und gegebenenfalls der weiteren Iodquellen kann auf einmal, in mehreren Portionen oder kontinuierlich erfolgen. Das Vermischen kann bei der gleichen Temperatur wie die Zugabe des Iods und gegebenenfalls der weiteren Iodquellen oder bei einer anderen, vorzugsweise einer höheren, Temperatur erfolgen.

Die Zugabe des Iods und gegebenenfalls der weiteren Iodquellen kann in Substanz, als Suspension oder Lösung, z. B. in Wasser oder organischen Lösungsmitteln, erfolgen.

Vorzugsweise wird wenigstens das Vermischen der Reaktanden, insbesondere des Polyvinylpyrrolidons und des Iods, bei einer Temperatur oberhalb von 50 °C durchgeführt.

Wenn das erfindungsgemäße Verfahren in Gegenwart eines Reduktionsmittels durchgeführt wird, kann die Zugabe ganz oder teilweise durch Vorvermischen von Reduktionsmittel und PVP und/oder durch Zugabe von Reduktionsmittel zur Vorlage und/oder durch Zugabe von Reduktionsmittel während des Vermischens erfolgen.

Unter Vermischen oder Mischen ist im Rahmen dieser Erfindung ein Vermengen oder Homogenisieren zu verstehen, das den Kontakt der der zu vermischenden Komponenten, hier auch als Reaktanden bezeichnet, erhöhen und damit eine gleichmäßige und/oder rasche Bildung des gewüschten Produktes ermöglichen soll. Durch das vermischen kann ein möglichst homogenes Gemisch erzeugt und/oder eine chemische Reaktion eingeleitet oder beschleunigt werden.

Methoden, die ein vermischen bewirken können sind beispielsweise Rühren, Schütteln, das Eindüsen von Gasen oder Flüssigkeiten und das Bestrahlen mit Ultraschall.

Geeignete Verfahren und Vorrichtungen, die ein Vermischen oder Mischen bewirken, sind dem Fachmann bekannt. Geeignete Mischvorrichtungen sind beispielsweise solche, die auch in der Kunststofftechnologie zum Mischen eingesetzt werden. Geeignete Vorrichtungen sind beispielsweise beschrieben in "Mischen beim Herstellen und Verarbeiten von Kunststoffen", H. Pahl, VDI-Verlag 1986. Geeignete Mischapparaturen sind beispielsweise Rührkessel, dynamische und statische Mischer, einwellige Rührwerke, beispielsweise Rührwerke mit Abstreifvorrichtungen, insbesondere sogenannte Pastenrührwerke, mehrwellige Rührwerke, insbesondere PDSM-Mischer, Feststoffmischer sowie Misch-/Knetreaktoren (z. B. ORP, CRP, AP, DTP der Firma List und Reaktotherm der Fa. Krauss-Maffei), Doppelmuldenkneter, Trogmischer und Stempelkneter (Innenmischer), Extruder, z. B. die ZKS-Modelle der Fa. Werner & Pfleiderer, sowie Rotor-Stator-Systeme, z. B. Dispax der Fa. Ika. Geeignete Extruder sind beispielsweise Einschnekkenmaschinen, kämmende Schneckenmaschinen oder auch Mehrwellenextruder, insbesondere Zweischneckenextruder, z. B. gleichsinnig oder gegensinnig drehende Zweischneckenextruder. Vorzugsweise werden Mischvorrichtungen verwendet, die beheizbar sind.

Das erfindungsgemäße Verfahren wird vorzugsweise bei einer Temperatur im Bereich von 10 bis 110 °C, bevorzugt im Bereich von 20 bis 105 °C durchgeführt. Vorzugsweise beträgt die Reaktionszeit beim erfindungsgemäßen Verfahren 20 Minuten bis 20 Stunden, vorzugsweise 30 Minuten bis 12 Stunden und besonders bevorzugt 1 Stunde bis 6,5 Stunden. Im Sinne dieser Erfindung ist Reaktionszeit als die Zeit zwischen dem ersten Kontakt von einer Iodquelle mit Polyvinylpyrrolidon in wässriger Lösung bis zum Vorliegen einer stabilen PVP-Iod-Lösung zu verstehen. Unter einer stabilen PVP-Iod-Lösung ist im Rahmen dieser Erfindung vorzugsweise eine PVP-Iod-Lösung zu verstehen, die einen Iodverlust in den vorstehend genannten Grenzen, bestimmt nach den vorgenannten Methoden, aufweist.

Vorzugsweise erwärmt man beim erfindungsgemäßen Verfahren das Gemisch über einen Zeitraum von 30 Minuten bis 15 Stunden, insbesondere 60 Minuten bis 12 Stunden und besonders bevorzugt 60 Minuten bis 6,5 Stunden auf eine Temperatur im Bereich von 50 bis 110 °C, insbesondere 70 bis 105 °C und ganz besonders bevorzugt im Bereich von 85 bis 100 °C.

Das Vermischen kann auch in Gegenwart eines Reduktionsmittels durchgeführt werden. Geeignete Reduktionsmittel sind insbesondere solche, die in der Lage sind, die Iodidbildung aus elementarem Iod zu beschleunigen.

Besonders geeignet sind solche Verbindungen, die nach der Reaktion mit Iod neben dem Iodid und gegebenenfalls Wasser ausschließlich flüchtige Stoffe, wie z. B. Kohlendioxid oder Stickstoff, bilden. Vorzugsweise ist das Reduktionsmittel ausgewählt unter Ameisensäure, Oxalsäure, den Estern und Salzen der Ameisen- und der Oxalsäure sowie den Amiden der Kohlensäure, der Ameisensäure und der Oxalsäure.

Besonders geeignete Reduktionsmittel sind beispielsweise Ammoniumcarbonat, Ammoniumhydrogencarbonat, Ammoniumcarbaminat, Harnstoff, Ammoniumformiat, Formamid, Ammoniumoxalat, Oxamidsäure und Oxamid. Besonders bevorzugt sind Oxalsäure und Ameisensäure sowie ihre Amide und Ammoniumsalze. Ganz besonders bevorzugt sind Oxalsäure und Ameisensäure, die bei der Reaktion mit Iod neben Iodwasserstoff nur noch Kohlendioxid bilden.

Die Menge an Reduktionsmittel wird in der Regel so bemessen, dass bei vollständiger Umsetzung 1/5 bis 2/5, bevorzugt etwa 1/3, des zugesetzten Iods zu Iodid umgesetzt wird. Die Menge liegt daher, berechnet auf das eingesetzte Iod, je nach Art des zusatzes zwischen 2 und 30 Gew.-%, bevorzugt 4 und 22 Gew.-%.

Vorzugsweise werden mit dem erfindungsgemäßen Verfahren Polyvinylpyrrolidon-Iod-Lösungen hergestellt, die einen Gehalt an verfügbarem Iod, d. h. mit Thiosulfat titrierbares Iod, im Bereich von 2 bis 40 Gew.-%, bevorzugt 4 bis 30 Gew.-% und besonders bevorzugt 7,5 bis 15 Gew.-%, bezogen auf das Gesamtgewicht von PVP-Iod, gerechnet als Feststoff aufweisen. Die mit diesem Verfahren erhaltenen PVP-Iod-Lösungen lassen sich praktisch beliebig verdünnen oder aufkonzentrieren, z. B. bis zu festem PVP-Iod.

Das erfindungsgemäße Verfahren soll im Folgenden anhand einiger Ausführungsformen näher erläutert werden:

Das erfindungsgemäße Verfahren kann beispielsweise so durchgeführt werden, dass man Wasser und gegebenenfalls Reduktionsmittel vorlegt, Iod und gegebenenfalls weitere Iodquellen hinzufügt und anschließend unter Rühren PVP-Lösung zugibt. Vorzugsweise wird nach Zugabe des Iods bzw. der Iodquellen das wässrige iodhaltige Reaktionsgemisch heftig vermischt, z. B. 10 Minuten bis 120 Minuten, bevor man die PVP-Lösung zugesetzt. Vorzugsweise wird das Reaktionsgemisch nach der Zugabe der PVP-Lösung innerhalb von 10 Minuten bis 2 Stunden, z. B. etwa 1 Stunde, auf 70 bis 100 °C, z. B. etwa 90 °C, erwärmt und anschließend 2 bis 10 Stunden weiter vermischt.

In einer bevorzugten Ausführungsform des erfindungsgemäßen Verfahrens legt man die Polyvinylpyrrolidon-Lösung und gegebenenfalls Reduktionsmittel vor, erwärmt auf eine Temperatur im Bereich von 50 bis 110 °C, bevorzugt 70 bis 105 °C und besonders bevorzugt auf eine Temperatur von etwa 85 °C oder etwa 100 °C, gibt bei dieser Temperatur elementares Iod und gegebenenfalls weitere Iodquellen zu und vermischt die Reaktanden 2 bis 12 Stunden, vorzugsweise 2,5 bis 10 Stunden und besonders bevorzugt 3 bis 6,5 Stunden. Die Zugabe des elementaren Iods und gegebenenfalls der weiteren Iodquellen kann in einer Portion, in 2 bis 15 Portionen, beispielsweise 5 oder 10 Portionen, oder kontinuierlich erfolgen. Wenn das Iod bzw. die Iodquellen in mehreren Portionen oder kontinuierlich zugegeben werden, erfolgt die Zugabe vorzugsweise über einen Zeitraum von 20 bis 90, vorzugsweise 30 bis 70 und besonders bevorzugt 40 bis 60 Minuten.

In einer besonders bevorzugten Ausführungsform des erfindungsgemäßen Verfahrens legt man eine PVP-Lösung eines Polyvinylpyrrolidons mit einem K-Wert > 27 und einer Konzentration c von etwa 45 Gew.-% und ein Reduktionsmittel, vorzugsweise Ameisensäure, vor und gibt anschließend elementares Iod in mehreren Portionen, vorzugsweise etwa 10 gleichen Portionen zu, wobei der Abstand zwischen den einzelnen Portionen einige Minuten, vorzugsweise etwa 5 bis 10 Minuten beträgt. Die Zugabe des Iods kann im Bereich der Raumtemperatur, z.B. etwa 25 °C, oder zu der auf eine Temperatur im Bereich von 40 bis 100 °C, z.B. etwa 50 °C oder etwa 85 °C, erwärmten Vorlage erfolgen. Nach beendeter Zugabe wird das Reaktionsgemisch weitere 2 bis 6 Stunden bei einer Temperatur im Bereich von 70 bis 100 °C, z. B. etwa 85 °C, vermischt. Anschließend kann das Reaktionsgemisch durch Zugabe von Wasser verdünnt werden, beispielsweise auf einen Feststoffgehalt im Bereich 5 bis 40 Gew.-%, wie etwa 20 Gew.-% oder etwa 30 Gew.-%.

Die nach diesem Verfahren erhältlichen Polyvinylpyrrolidon-Iod-Lösungen weisen vorzugsweise ein Gehalt an verfügbarem Iod von wenigstens 4 Gew.-% auf, bezogen auf das Polyvinylpyrrolidon-Iod, gerechnet als Feststoff.

Die erfindungsgemäß hergestellten PVP-Iod-Lösungen können direkt auf die übliche Art, z. B. unter Zugabe von weiteren Hilfsstoffen, beispielsweise Tensiden, zu für den Verbraucher bestimmten Endprodukten formuliert werden. Diese Lösungen haben im Allgemeinen eine Gesamtkonzentration von 10 bis 50 Gew.-% an Feststoffen.

Weiterer Gegenstand der vorliegenden Erfindung ist eine Polyvinylpyrrolidon-Iod-Lösung, erhältlich nach einem wie vorstehend beschriebenen Verfahren. Die erfindungsgemäßen PVP-Iod-Lösungen weisen vorzugsweise ein Polyvinylpyrrolidon mit einem K-Wert von 25 bis 90 und insbesondere 25 bis 35 und einen Feststoffgehalt an PVP-Iod im Bereich von 25 bis 55 Gew.-%, insbesondere 30 bis 50 Gew.-% auf. Vorteilhaft weisen die erfindungsgemäßen PVP-Iod-Lösungen einen Iodverlust von kleiner als 4 %, bestimmt nach 15h bei 80 °C, und einen Gehalt an freiem Iod (flüchtigem Iod) von kleiner 2 ppm auf.

Weiterer Gegenstand der vorliegenden Erfindung ist eine festes Polyvinylpyrrolidon-Iod, erhältlich durch Entfernen des Wassers und anderer flüchtiger Bestandteile aus einer wässrige Polyvinylpyrrolidon-Iod-Lösung, wie zuvor definiert.

Weiterer Gegenstand der vorliegenden Erfindung ist die Verwendung einer wässrigen Polyvinylpyrrolidon-Iod-Lösung oder von festem Polyvinylpyrrolidon-Iod, wie zuvor definiert, zur Herstellung von Mitteln zur Desinfektion, Feindesinfektion oder zur Wundbehandlung sowie zur Herstellung von Wundauflagen.

Weiterer Gegenstand der vorliegenden Erfindung ist ein Feindesinfektionsmittel, enthaltend eine wässrige Polyvinylpyrrolidon-Iod-Lösung oder festes Polyvinylpyrrolidon-Iod, wie vorstehend beschrieben.

Ein weiterer Vorteil der erfindungsgemäß erhältlichen Polyvinylpyrrolidon-Iod-Lösung ist, dass diese direkt als Lösung eingesetzt werden können und nicht erst das PVP-Iod aus der wässrigen Lösung isoliert werden muss, da die erfindungsgemäß hergestellten PVP-Iod-Lösungen in vorteilhaft hohen Konzentrationen anfallen. Daher müssen diese in der Regel nicht aufwendig aufkonzentriert werden, sondern können, gegebenenfalls nach Verdünnen, direkt zu gebrauchsfertigen PVP-Lösungen formuliert werden. Die hohe Konzentration der erfindungsgemäß hergestellten PVP-Iod-Lösungen ist auch vorteilhaft, wenn daraus durch Entfernen des Wassers und anderer flüchtiger Bestandteile festes Polyvinylpyrrolidon-Iod, beispielsweise Polyvinylpyrrolidon-Iod-Pulver oder -Granulate, hergestellt werden sollen, da weniger flüchtige Bestandteile, insbesondere Wasser, entfernt werden müssen. Die nach diesem Verfahren erhältlichen neuen Polyvinylpyrrolidon-Iod-Lösungen besitzen überraschenderweise trotz der verringerten Reaktionszeit eine höhere Stabilität als herkömmliche Lösungen, wie insbesondere aus einem Iodverlust < 5 % ersichtlich ist.

Festes PVP-Iod kann gewünschtenfalls aus den erfindugsgemäß erhaltenen PVP-Iod-Lösungen erhalten werden, beispielsweise durch Ausfällen des PVP-Iods aus der Lösung und gegebenenfalls anschliesendes Abfiltrieren, oder durch einen Trockenprozess, beispielsweise durch Gefriertrocknung, Walzentrocknung, Sprühtrocknung oder Sprühgranulierung.

Die folgenden Beispiele erläutern die Erfindung, ohne sie zu beschränken. Die K-Werte wurden nach H. Fikentscher (siehe oben) bestimmt. Die Bestimmung des Iodverlustes (JV) erfolgte durch 15-stündige Lagerung einer wässrigen PVP-Iod-Lösung mit einem Gehalt an verfügbarem Iod von 1 % bei einer Temperatur von 80 °C. Der Verteilungskoeffizient (VK) wurde gemäß US-A-3,028,300 durch 1-minütiges intensives Schütteln von 1,0 ml einer wässrigen PVP-Iod-Lösung mit einem verfügbaren Iodgehalt von 1,0 % mit 25 ml Heptan in einem verschlossenen Glaskolben in einem thermostatisierten Heizbad von 25 °C bestimmt. Nach einigen Minuten Stehen wurden die beiden Phasen getrennt und der Iodgehalt der wässrigen Phase durch Titration mit Natriumthiosulfat und der Iodgehalt der Heptanphase spektralphotometrisch bestimmt. Die Berechnung erfolgte gemäß folgender Gleichung:

$$VK = \frac{\text{mg Iod in H}_2\text{O-Phase}}{\text{mg Iod in Heptan}} \times \frac{\text{ml Heptan (25)}}{\text{ml H}_2\text{O-Phase (1)}}$$

Beispiele

Die analytischen Daten des Vergleichsbeispiels (VB1) und der erfindungsgemäßen Beispiele (B2 bis B9) sind in Tabelle 1 zusammengefasst.

Vergleichsbeispiel 1

In einem Rührreaktor wurden 1140 g einer 17,5 gew.-%iqen Polyvinylpyrrolidon-Lösung eines Polyvinylpyrrolidons mit K-Wert 30 und 1,86 g Ameisensäure vorgelegt. Anschließend wurden unter Rühren 35 g elementares Iod in 10 gleichen Portionen in Abständen von etwa 5 Minuten zugegeben. Anschließend wurde das Reaktionsgemisch unter Rühren auf 70 °C erwärmt und weitere 20 Stunden bei dieser Temperatur gerührt. Das Reaktionsgemisch wies eine uneinheitliche Viskosität aufgrund von Verbackungen auf.

Beispiel 2

In einem Rührkessel wurden 733 g Polyvinylpyrrolidon-Lösung eines Polyvinylpyrrolidons mit einem K-Wert von 30 und einer Konzentration c von 45 Gew.-% und 3,72 g Ameisensäure vorgelegt und auf 100 °C erwärmt. Anschließend wurden unter Rühren 70 g elementares Iod in 10 gleichen Portionen in Abständen von etwa 5 Minuten zugegeben. Danach wurde weitere 10 Stunden bei dieser Temperatur gerührt. Anschließend wurde das Reaktionsgemisch mit Wasser auf einen Feststoffgehalt von 30,0 Gew.-% verdünnt.

Beispiel 3

In einem Rührkessel wurden 1,369 kg Polyvinylpyrrolidon-Lösung eines Polyvinylpyrrolidons mit einem K-Wert von 30 und einer Konzentration c von 45 Gew.-% und 6,95 g Ameisensäure vorgelegt und unter Rühren auf 85 °C erwärmt. Anschließend wurden unter Rühren 131 g elementares Iod in 10 gleichen Portionen in Abständen von etwa 5 Minuten zugegeben. Danach wurde das Reaktionsgemisch noch 7,5 Stunden bei 85 °C gerührt und anschließend mit Wasser auf einen Feststoffgehalt von 30 Gew.-% verdünnt.

Beispiel 4

In einem Rührkessel wurden 733 g Polyvinylpyrrolidon-Lösung eines Polyvinylpyrrolidons mit einem K-Wert von 30 und einer Konzentration c von 45 Gew.-% und 3,72 g Ameisensäure vorgelegt und auf 85 °C erwärmt. Anschließend wurden unter Rühren 70 g elementares Iod in 10 gleichen Portionen in Abständen von etwa 5 Minuten zugegeben. Danach wurde weitere 6 Stunden bei dieser Temperatur gerührt. Anschließend wurde das Reaktionsgemisch mit Wasser auf einen Feststoffgehalt von 30,0 Gew.-% verdünnt.

Beispiel 5

Beispiel 5 wurde analog zu Beispiel 4 in einer Technikumsanlage mit einem Scale-up-Faktor von 130 durchgeführt.

Beispiel 6

Beispiel 6 wurde analog Beispiel 4 durchgeführt, jedoch wurde nach Beendigung der Iodzugabe 6 Stunden bei 90 °C gerührt.

Beispiel 7

In einem Rührkessel wurden 733 g Polyvinylpyrrolidon-Lösung eines Polyvinylpyrrolidons mit einem K-Wert von 30 und einer Konzentration c von 45 Gew.-% und 3,72 g Ameisensäure vorgelegt und unter Rühren auf 50 °C erwärmt. Anschließend wurden unter Rühren 70 g elementares Iod in 10 gleichen Portionen in Abständen von etwa 5 Minuten zugegeben. Danach wurde das Reaktionsgemisch unter Rühren rasch auf 85 °C erwärmt, weitere 6 Stunden bei 85 °C gerührt und anschließend mit Wasser auf einen Feststoff gehalt von 30 Gew.-% verdünnt.

Beispiel 8

Beispiel 8 wurde analog Beispiel 7 duschgeführt, jedoch erfolgte die Iod-Zugabe bei 25 °C und es wurde nur 4 Stunden bei 85 °C gerührt.

Beispiel 9

Beispiel 8 wurde analog Beispiel 8 durchgeführt, jedoch wurde nur 3 Stunden bei 85 °C gerührt.
Bei keinem der erfindungsgemäßen Beispiele B2 bis B9 wurde die Bildung eines Bodensatzes beobachtet. Die erfindungsgemäßen Beispiele B2 bis B9 wiesen alle Verteilungskoeffizienten VK im Bereich von etwa 200 oder darüber auf und erfüllten die spezifikationen für die Verwendung in Pharmazeutika.

Tabelle 1:

| | PVP-Lösung | | | Red.-Mittel[1] [g] | Iod [kg] | Reaktionstemperatur[2] [°C] | Reaktionszeit [Stunden] | PVP-Iod-Lösung | | | |
|---|---|---|---|---|---|---|---|---|---|---|---|
| | Menge [kg] | c [Gew.-%] | K-Wert | | | | | FS vor verdünnen[3] | FS nach verdünnen[3] | Verf. Iod[4] [Gew.-%] | Iod-Verlust[5] [Gew.-%] |
| VB1 | 1,140 | 17,5 | 30 | 1,86 | 0,035 | 25 / 70 | 21 | 19,6 | – | 9,68 | 4,38 |
| B2 | 0,733 | 45 | 30 | 3,72 | 0,070 | 100 | 11 | ca. 50 | 30,0 | 10,0 | 4,8 |
| B3 | 1,369 | 45 | 30 | 6,95 | 0,131 | 100 | 8,5 | ca. 50 | 30,0 | 10,9 | 4,6 |
| B4 | 0,733 | 45 | 30 | 3,72 | 0,070 | 85 | 7 | ca. 50 | 30,0 | 11,9 | 1,7 |
| B5[6] | 178,1 | 45 | 30 | 903 | 17,03 | 85 | 7 | ca. 50 | 29,5 | 10,8 | 3,6 |
| B6 | 0,733 | 45 | 30 | 3,72 | 0,070 | 85 / 90 | 7 | ca. 50 | 30,2 | 11,4 | 2,6 |
| B7 | 0,733 | 45 | 30 | 3,72 | 0,070 | 50 / 85 | 7 | ca. 50 | 29,9 | 11,6 | 2,6 |
| B8 | 0,733 | 45 | 30 | 3,72 | 0,070 | 25 / 85 | 5 | ca. 50 | 29,9 | 12,25 | 3,3 |
| B9 | 0,733 | 45 | 30 | 3,72 | 0,070 | 25 / 85 | 4 | ca. 50 | 30,3 | 11,86 | 3,4 |

1) Ameisensäure, 100%ig

2) Bei Angabe zweier Werte bezieht sich der erste Wert auf die Temperatur bei der Iod-Zugabe

3) FS steht für den Feststoffgehalt in Gew.-%

4) Bestimmt durch Titration mit Thiosulfat

5) Bestimmt nach 15-stündigem erwärmem einer 1%-tigen Lösung auf 80 °C

6) Technikums-Versuch

EP 1 211 942 B1

**Patentansprüche**

1. Verfahren zur Herstellung von Polyvinylpyrrolidon-Iod in wässriger Lösung, wobei man eine wässrige Polyvinylpyrrolidon-Lösung und wenigstens 4,0 Gew.-% elementares Iod, bezogen auf das als Feststoff gerechnete Polyvinylpyrrolidon, vermischt, **dadurch gekennzeichnet, dass** zum Zeitpunkt des Vermischens die Konzentration c des wässrigen Polyvinylpyrrolidons, bezogen auf die Gesamtmenge aus Polyvinylpyrrolidon und Wasser, und der K-Wert des Polyvinylpyrrolidons der folgenden Relation gehorcht:

$$c > 100 \times [0{,}1 + 8 : (K + 5)]$$

wobei c in Gew.-% angegeben ist und der R-Wert nach Fikentscher im Bereich von 10 bis 100 liegt.

2. Verfahren nach Anspruch 1, **dadurch gekennzeichnet, dass** man das Gemisch über einen Zeitraum von 30 Minuten bis 15 Stunden auf eine Temperatur im Bereich von 50 bis 110 °C erwärmt.

3. Verfahren nach Anspruch 1 oder 2, **dadurch gekennzeichnet, dass** man das Vermischen in Gegenwart eines Reduktionsmittels durchführt.

4. Verfahren nach einem der vorhergehenden Ansprüche, **dadurch gekennzeichnet, dass** das Reduktionsmittel ausgewählt ist unter Ameisensäure, Oxalsäure, den Estern und Salzen der Ameisen- und der Oxalsäure sowie den Amiden der Kohlensäure, der Ameisensäure und der Oxalsäure.

5. Verfahren nach einem der vorhergehenden Ansprüche, **dadurch gekennzeichnet, dass** man die Polyvinylpyrrolidon-Lösung und gegebenenfalls wenigstens einen Teil des Reduktionsmittels vorlegt, das Gemisch gegebenenfalls erwärmt und anschließend Iod zugibt.

6. Verfahren nach einem der vorhergehenden Ansprüche, **dadurch gekennzeichnet, dass** man eine Polyvinylpyrrolidon-Lösung eines Polyvinylpyrrolidons mit einem K-Wert > 27 und einem Polyvinylpyrrolidon-Gehalt von > 35 Gew.-% einsetzt.

7. Verfahren nach einem der vorhergehenden Ansprüche, **dadurch gekennzeichnet, dass** das in der Lösung enthaltene Polyvinylpyrrolidon-Iod einen Gehalt an verfügbarem Iod von wenigstens 4 Gew.-% aufweist.

8. Polyvinylpyrrolidon-Iod-Lösung, erhältlich nach einem Verfahren gemäß einem der Ansprüche 1 bis 7.

9. Festes Polyvinylpyrrolidon-Iod, erhältlich durch Entfernen des Wassers und anderer flüchtiger Bestandteile aus einer wässrigen Polyvinylpyrrolidon-Iod-Lösung, wie in Anspruch 8 definiert.

10. Verwendung einer wässrigen Polyvinylpyrrolidon-Iod-Lösung wie in Anspruch 8 definiert oder von festem Polyvinylpyrrolidon-Iod wie in Anspruch 9 definiert, zur Herstellung von Mitteln zur Desinfektion, Feindesinfektion oder zur Wundbehandlung.

11. Verwendung nach Anspruch 10 zur Herstellung von Wundauflagen.

12. Feindesinfektionsmittel, enthaltend eine wässrige Polyvinylpyrrolidon-Iod-Lösung wie in Anspruch 8 definiert oder festes Polyvinylpyrrolidon-Iod wie in Anspruch 9 definiert.

**Claims**

1. A process for preparing polyvinylpyrrolidone-iodine in aqueous solution, where an aqueous polyvinylpyrrolidone solution and at least 4.0% by weight of elemental iodine, based on the polyvinylpyrrolidone calculated as solid, are mixed, wherein at the time of mixing the concentration c of the aqueous polyvinylpyrrolidone, based on the total amount of polyvinylpyrrolidone and water, and the K value of polyvinylpyrrolidone obeys the following relation:

$$c > 100 \times [0.1 + 8 : (K + 5)]$$

where c is stated in % by weight, and the Fikentscher K value is in the range from 10 to 100.

2. A process as claimed in claim 1, wherein the mixture is heated at a temperature in the range from 50 to 110°C for a period of from 30 minutes to 15 hours.

3. A process as claimed in claim 1 or 2, wherein the mixing is carried out in the presence of a reducing agent.

4. A process as claimed in any of the preceding claims, wherein the reducing agent is selected from formic acid, oxalic acid, the esters and salts of formic and oxalic acids, and the amides of carbonic acid, of formic acid and of oxalic acid.

5. A process as claimed in any of the preceding claims, wherein the polyvinylpyrrolidone solution and, where appropriate, at least part of the reducing agent are mixed, the mixture is heated where appropriate, and then iodine is added.

6. A process as claimed in any of the preceding claims, wherein a polyvinylpyrrolidone solution of a polyvinylpyrrolidone with a K value of > 27 and a polyvinylpyrrolidone content of > 35% by weight is employed.

7. A process as claimed in any of the preceding claims, wherein the polyvinylpyrrolidone-iodine present in the solution has an available iodine content of at least 4% by weight.

8. A polyvinylpyrrolidone-iodine solution obtainable by a process as claimed in any of claims 1 to 7.

9. A solid polyvinylpyrrolidone-iodine obtainable by removing the water and other volatile constituents from an aqueous polyvinylpyrrolidone-iodine solution as defined in claim 8.

10. The use of an aqueous polyvinylpyrrolidone-iodine solution as defined in claim 8 or of solid polyvinylpyrrolidone-iodine as defined in claim 9 for producing compositions for disinfection, antisepsis or for wound treatment.

11. The use as claimed in claim 10 for producing wound coverings.

12. An antiseptic composition comprising an aqueous polyvinylpyrrolidone-iodine solution as defined in claim 8 or solid polyvinylpyrrolidone-iodine as defined in claim 9.

**Revendications**

1. Procédé pour la préparation du polyvinylpyrrolidone-iode en solution aqueuse selon lequel on mélange une solution aqueuse de polyvinylpyrrolidone et au moins 4,0 % en poids d'iode élémentaire par rapport aux matières solides de la polyvinylpyrrolidone, ce procédé se caractérisant par le fait que, au moment du mélange, la concentration c de la polyvinylpyrrolidone aqueuse, rapportée à la quantité totale de polyvinylpyrrolidone et d'eau, et l'indice K de la polyvinylpyrrolidone satisfont à la relation suivante

$$c > 100 \times [0{,}1 + 8 : (K + 5)]$$

dans laquelle c est exprimé en % en poids et l'indice K selon Fikentscher se situe dans l'intervalle de 10 à 100.

2. Procédé selon la revendication 1, **caractérisé par le fait que** l'on chauffe le mélange pendant une durée de 30 minutes à 15 h à une température dans l'intervalle de 50 à 110°C.

3. Procédé selon la revendication 1 ou 2, **caractérisé par le fait que** l'on procède au mélange en présence d'un agent réducteur.

4. Procédé selon l'une des revendications qui précèdent, **caractérisé par le fait que** l'agent réducteur est choisi dans le groupe formé par l'acide formique, l'acide oxalique, les esters et sels de l'acide formique et de l'acide oxalique et les amides de l'acide carbonique, de l'acide formique et de l'acide oxalique.

**5.** Procédé selon l'une des revendications qui précèdent, **caractérisé par le fait que** l'on mélange la solution de polyvinylpyrrolidone et le cas échéant une partie au moins de l'agent réducteur, on chauffe éventuellement le mélange puis on ajoute l'iode.

**6.** Procédé selon l'une des revendications qui précèdent, **caractérisé par le fait que** l'on utilise une solution d'une polyvinylpyrrolidone d'indice K supérieur à 27 et à une teneur en polyvinylpyrrolidone supérieure à 35 % en poids.

**7.** Procédé selon l'une des revendications qui précèdent, **caractérisé par le fait que** le polyvinylpyrrolidone-iode contenu dans la solution a une teneur en iode disponible d'au moins 4 % en poids.

**8.** Solution de polyvinylpyrrolidone-iode obtenue par un procédé selon l'une des revendications 1 à 7.

**9.** Polyvinylpyrrolidone-iode solide obtenu par élimination de l'eau et des autres constituants volatils d'une solution aqueuse de polyvinylpyrrolidone-iode telle que définie dans la revendication 8.

**10.** Utilisation d'une solution aqueuse de polyvinylpyrrolidone-iode telle que définie dans la revendication 8 ou d'un polyvinylpyrrolidone-iode solide tel que défini dans la revendication 9 pour la préparation de produits pour désinfection, désinfection fine ou traitement des blessures.

**11.** Utilisation selon la revendication 10 pour la préparation de pansements pour blessures.

**12.** Agent désinfectant fin contenant une solution aqueuse de polyvinylpyrrolidone-iode telle que définie dans la revendication 8 ou un polyvinylpyrrolidone-iode solide tel que défini dans la revendication 9.